# EUROPEAN PATENT APPLICATION

(11) **EP 4 755 465 A1**
(43) Date of publication of application: **10.06.2026**
(21) Application number: 25220839.2
(22) Date of filing: 04.12.2025
(51) Int. Cl.: A61Q 1/10, A61K 8/26, A61K 8/31, A61K 8/41, A61K 8/49, A61K 8/73, A61K 8/891, A61K 8/92

(54) **ANHYDROUS COSMETIC COMPOSITION**

(30) Priority: 05.12.2024 US 202463728638 P
(71) Applicant: Shiseido Company, Limited, Chuo-Ku Tokyo 104-0061 (JP)
(72) Inventor: DANYANSAH, Nana, Perth Amboy, 08861 (US); SATURNE-NICOLAS, Fabienne, Franklin Park, 08823 (US)
(74) Representative: Ter Meer Steinmeister & Partner

(57) **Abstract**

Provided is an anhydrous cosmetic composition and methods for its using and making. The composition includes a thickening system that includes a silicone elastomer, disteardimonium hectorite and waxes. The waxes include sorbitan olivate, microcrystalline wax and dextrin palmitate. The composition may be used, for example, as an eyeshadow base.

## Description

### Field

The present application relates to cosmetic compositions and related methods of making and use and, more specifically, to anhydrous cosmetic compositions and their methods of making and use.

### SUMMARY

One embodiment is an anhydrous cosmetic composition comprising (a) one or more oils; (b) a thickening system comprising a silicone elastomer, disteardimonium hectorite and waxes; and (c) one or more powders, said waxes comprise sorbitan olivate, microcrystalline wax and dextrin palmitate.

### FIGURES

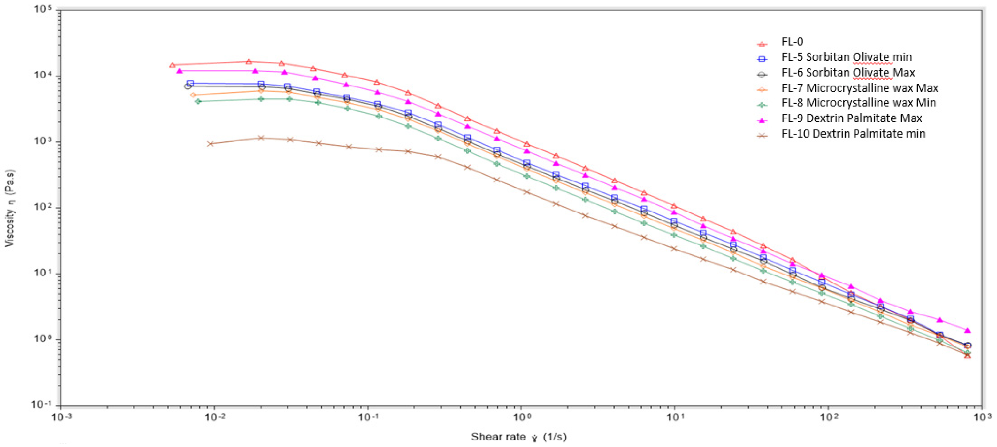

The Figure shows a shear viscosity profile for exemplary compositions.

### DETAILED DESCRIPTION

Unless otherwise specified "a" or "an" means one or more.

As used herein, the term "about" placed before a specific numeric value may mean ±20% of the numeric value; ± 18% of the numeric value, ± 15% of the numeric value; ± 12% of the numeric value; ±8% of the numeric value; ±5% of the numeric value; ±3% of the numeric value; ±2% of the numeric value; ±1% of the numeric value or ±0.5% of the numeric value. All the numeric values in this disclosure other than the numeric values in the working examples should be treated as preceded by the word "about."

Unless otherwise specified, all content information for ingredients of compositions expressed as % refers to % by mass, relative to the total mass of the composition.

As used herein, the expression "anhydrous composition" means that the composition contains no more than 2 mass % of water, no more than 1 mass %, no more 0.5 mass % of water; no more than 0.2 mass % of water, no more than 0.1 mass % of water, no more than 0.05 mass % of water, no more than 0.02 mass % of water, or no more than 0.01 mass % of water. In many embodiments, the composition does not contain any water at all.

An eyeshadow base composition is a cosmetic composition that may smoothen and intensify an eyeshadow for day-to-night lasting color. It is desirable to have an eyeshadow base composition that is smudge-proof, long lasting and has an invisible finish. It is also desirable that an eyeshadow base composition could work on all skin tones, while being never drying. Furthermore, it is desirable for an eyeshadow base composition to be lightweight and crease-proof. As used herein, the term "smudge-proof" may mean the composition stays in place after it sets. The smudge-proof composition is not easily removed and/or altered (in aesthetical appearance) while being worn during typical activities. As used herein, the term "long lasting" may mean that the composition stays on a keratinous surface or substrate, such as skin, for at least 4 hours or at least 6 hours or at least 8 hours or at least 10 hours or at least 12 hours or at least 14 hours or at least 16 hours or at least 18 hours or at least 20 hours without interruption of esthetical appearance. As used herein, the term "invisible finish" may refer to the situation when the composition is used in combination with another color cosmetic composition (as a primer applied to the skin first and then the other cosmetic composition is applied on top of it), and the composition does not change the color appearance of the other color cosmetic composition. For example, there is no white cast after applying the cosmetic composition of this application. As used herein, "all skin tones" means that the cosmetic composition can be used for lighter skin tones as well as for darker skin tones. As used herein, "never drying" may mean that the cosmetic composition does not provide feeling of dryness when applied to a keratinous surface or substrate, such as skin. As used herein, "lightweight" means that the cosmetic composition does not feel heavy when applied to a keratinous surface or substrate, such as skin. As used herein, "crease-proof" means that the composition is smooth without forming creases when applied to a keratinous surface or substrate, such as skin.

Currently available eyeshadow base compositions usually include talc and a volatile cyclomethicone (D4-D6) silicone solvent, such as decamethylcyclopentasiloxane (D5).

In general, talc can give a cosmetic product, such as an eyeshadow base composition, a nice payoff, transparency and texture. A volatile cyclomethicone (D4-D6) silicone solvent, such as decamethylcyclopentasiloxane, can give a cosmetics product, such as an eyeshadow base composition, a silk smooth and velvety texture while giving the skin a luxurious feel. As used herein, "transparency" may mean that the cosmetic composition has the invisible finish as discussed above. In other words, "transparency" may mean that the composition is being able to transmit visible light without scattering so that an underlying body can be seen clearly through the composition. As used herein, the term "payoff" may refer to the amount of the cosmetic composition applied to a keratinous surface or substrate, such as skin.

Many of currently available eyeshadow base compositions also include large amounts of disteardimonium hectorite (Bentone).

Disteardimonium hectorite can provide a cosmetic product, such as an eyeshadow base composition, with a viscosity control, while enhancing skin-feel by masking greasy or tacky ingredients and imparting a pleasant silkiness to the skin.

For regulatory reasons, it may be desirable to remove talc, a volatile cyclomethicone (D4-D6) silicone solvent, such as decamethylcyclopentasiloxane, and an excessive amount of disteardimonium hectorite from a cosmetic product, such as an eyeshadow base composition.

However, replacing talc and a volatile cyclomethicone (D4-D6) silicone solvent, such as decamethylcyclopentasiloxane, and avoiding an excessive amount of disteardimonium hectorite, while achieving the same texture and performance, may be a challenging task.

The present disclosure addresses such challenges.

The present disclosure provides an anhydrous cosmetic composition that nay include a thickener comprising a silicone elastomer, disteardimonium hectorite and waxes. The waxes may include sorbitan olivate, microcrystalline wax and dextrin palmitate.

The cosmetic composition may contain no talc, no volatile cyclomethicone (D4-D6) silicone solvent, such as decamethylcyclopentasiloxane, while containing a reduced amount of disteardimonium hectorite compared to existing compositions, while providing the same or similar the same texture and performance as the existing compositions.

The cosmetic composition may contain less than 0.1 mass % or less than 0.05 mass % or less than 0.02 mass % of volatile cyclomethicone (D4-D6) silicone solvent or may contain no volatile cyclomethicone (D4-D6) silicone solvent at all.

The cosmetic composition may contain less than 0.1 mass % or less than 0.05 mass % or less than 0.02 mass % of talc and may contain no talc at all.

In some embodiments, the composition may have a shear viscosity at 0.01 s⁻¹ from about 1000 Pa·s to about 40000 Pa·s, about 1000 Pa·s to about 20000 Pa·s, or about 3000 Pa·s to about 20000 Pa·s.

In some embodiments, the composition may have a shear viscosity at 1000 s⁻¹ from about 0.5 Pa·s to about 100 Pa·s, about 1 Pa·s to about 100 Pa·s or about 0.5 Pa·s to about 10 Pa·s.

In some embodiments, the cosmetic composition may feel lightweight when applied to a keratinous surface, such as a skin.

In some embodiments, the cosmetic composition may blend smoothly with a keratinous surface, such as a skin, after applying the composition to the keratinous surface.

The cosmetic composition may have texture comparable to commercially available cosmetic compositions containing talc and cyclomethicone (D4-D6) silicone solvent, such as decamethylcyclopentasiloxane.

In some embodiments, the cosmetic composition may be used alone. In other words, the composition may be applied alone, without another composition, to a keratinous surface or substrate, such as skin or hair, of a subject, such as a human. Yet, in some embodiments, the cosmetic composition may be used together with another composition, which may be, for example, a cosmetic or skincare composition, such as a makeup composition or another skin care product. For example, in certain embodiments, the cosmetic composition may be applied to the keratinous surface or substrate before or after another composition is applied.

Preferably, the cosmetic composition can be used as a primer, meaning that it may be deposited on the keratinous substrate as an initial product for delivering hydrating, plumping, blurring and/or long wear effect to the keratinous surface or substrate.

In some embodiments, the cosmetic composition may have one or multiple of unique sensory properties, such as those described above. In some embodiments, the cosmetic composition may provide a weightlessness, e.g., no heavy or sticky feel. In some embodiments, the cosmetic composition may be long lasting. For example, the cosmetic composition may stay on a keratinous surface or substrate for at least 4 hours or at least 6 hours or at least 8 hours or at least 10 hours or at least 12 hours or at least 14 hours or at least 16 hours or at least 18 hours or at least 20 hours without interruption of esthetical appearance.

In many embodiments, the cosmetic composition may be an eyeshadow base composition. In some embodiments, the cosmetic composition may be an under-eye composition, a cheek composition or a lip composition.

In some embodiments, the cosmetic composition may be a primer composition, such as an under-eye primer composition, a cheek primer composition or a lip primer composition.

The present composition may result in no smudging, creasing, and/or caking, when applied to a keratinous surface, such as a skin. The present composition may hold a color for at least 4 hours or at least 6 hours or at least 8 hours or at least 10 hours or at least 12 hours or at least 14 hours or at least 16 hours or at least 18 hours or at least 20 hours without interruption of esthetical appearance.

The texture of the present composition when used as an eyeshadow base composition may be comparable to commercial eyeshadow base compositions

In many embodiments, the composition may be a stable composition. As used herein, the term "stable" may mean that the composition stays homogeneous after storing at one or more or each of the following conditions: 4 weeks at 50 °C, 12 weeks at 45 °C, and 12 weeks at -5 °C. As used herein, the term "homogeneous" may mean that the composition exhibits no air cavities, no crystallization of waxes, no powder separation, no droplets of oil separating (sweating) after the storing.

### Silicone elastomer

The silicone elastomer may be a part of the thickening system of the composition.

In some embodiments, the silicone elastomer may be a silicone polymer. In some embodiments, the silicone elastomer may comprise a silicone polymer disclosed in U.S. Patent Application Publications Nos. 2007/0243143 and 2019/0350835, each of which is incorporated by reference in its entirety.

In some embodiments, the silicone elastomer may include a backbiting siloxane polymer disclosed in U.S. Patent Application Publication No. 2007/0243143.

The backbiting siloxane polymer may be generally synthesized by reacting a siloxane precursor such as a silicone macro monomer having a molecular weight of about 1,000 or more, preferably from about 1,000 to 5,000. Typically, the siloxane macro monomer contains a reactive group that permits chain propagation at one terminus. Examples of such reactive groups include vinyl, hydrido, epoxy, acrylate, acetoxy, or alkoxy groups and the like. Also suitable is where the silicone macro monomer used as the starting material contains organic oligomeric or polymeric portions containing terminal polyolefins, polyolefinic ethers, acrylates, epoxides, and the like. If desired, the silicone macromonomer used to synthesis the Backbiting polymer may be purchased rather than synthesized. For example, such macromonomers may be purchased from Sigma-Aldrich and include dimethylethoxyvinylsilane, vinyltrimethoxysilane, vinyltrimethylsilane, vinyltriethoxysilane, vinyltrichlorosilane, and so on. Alternatively, a silicon hydride can be treated with an olefinic compound such as butadiene to provide a monomer having a reactive group with olefinic unsaturation.

The backbiting occurs when the chain transfer is between two sites within the polymeric chain.

In some embodiments, a backbiting silicone polymer may conform to what is referred to as a linear to linear polymer structure and has the general formula: Such silicones have the following general formula: 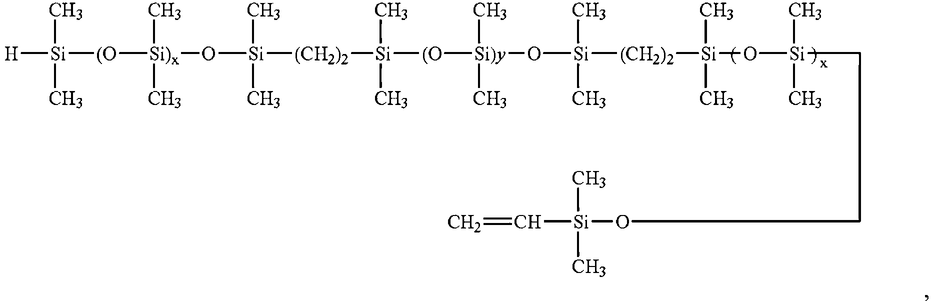 wherein x is 1 to 1,000,000.

Backbiting Siloxane Polymers include those from Jeen International Corporation sold under the tradenames JEESILC PS-CM, having the INCI name cyclomethicone, bis-vinyl dimethicone/dimethicone copolymer; JEESILC PS-CMLV, having the INCI name cyclomethicone, bis-vinyl dimethicone/dimethicone copolymer; JEESILC PS-DMLV, having the INCI name dimethicone, bis-vinyl dimethicone/dimethicone copolymer; JEESILC PS-VH, having the INCI name isododecane, bis-vinyl dimethicone/dimethicone copolymer; or JEESILC PS-VHLV, having the INCI name isododecane, bis-vinyl dimethicone/dimethicone copolymers.

In some embodiments, the silicone elastomer may include an elastomer having silicone chains crosslinked with silicone and exhibiting a structural viscosity when swollen in oil. Specific examples may include (dimethicone/vinyl dimethicone) crosspolymer and the like as defined by the nomenclature of cosmetic ingredients (INCI). These elastomers are commercially available as swollen products containing silicone oil or other oils and marketed under the trade name of KSG-15, 1510, 16, 15AP, and 19 (all from Shin-Etsu Chemical Co., Ltd.), for example.

Examples of the crosslinked product having alkyl branches on the main chain (alkyl-modified, partially crosslinked dimethylpolysiloxane, and silicone/alkyl-modified, partially crosslinked dimethylpolysiloxane), and the crosslinked product having a phenyl group in a crosslinked portion (phenyl-modified, partially crosslinked dimethylpolysiloxane) include (vinyl dimethicone/lauryl dimethicone) crosspolymer, (lauryl polydimethylsiloxyethyl dimethicone/bisvinyl dimethicone) crosspolymer, and (dimethicone/phenyl vinyl dimethicone) crosspolymer as defined by INCI. They are commercially available as swollen products containing normally liquid oil and marketed under the trade name of KSG-18A, 41A, 42A, 43, 44, 042Z, 045Z, 048Z, and 18A (all from Shin-Etsu Chemical Co., Ltd.), for example.

In some embodiments, the silicone elastomer may include a commercially available silicone elastomer, such as DOWSIL^{™} EL-9241, commercially available from Dow Corning Inc. DOWSIL^{™} EL-9241 has an INCI Name: Dimethicone & Dimethicone Crosspolymer.

An amount of the silicone elastomer, such as DOWSIL^{™} EL-9241, in the composition may vary. For example, in some embodiments, the composition may contain from 3 mass % to 12 mass % or from 5 mass % to 12 mass % or from 8 mass % to 12 mass % or from 9 mass % to 11.5 mass % of the silicone elastomer, such as DOWSIL^{™} EL-9241.

### Waxes

Waxes, including sorbitan olivate, microcrystalline wax and dextrin palmitate, may be a part of the thickening system of the composition.

Examples of additional waxes include acacia, beeswax, ceresin, cetyl esters, flower wax, citrus wax, carnauba wax, jojoba wax, japan wax, polyethylene, rice bran, lanolin wax, mink, montan, bayberry, ouricury, ozokerite, palm kernel wax, paraffin, avocado wax, apple wax, shellac wax, clary wax, spent grain wax, candelilla, grape wax, and polyalkylene glycol derivatives thereof such as PEG6-20 beeswax, or PEG-12 carnauba wax; or fatty acids or fatty alcohols, including esters thereof, such as hydroxystearic acids (for example 12-hydroxy stearic acid), tristearin, tribehenin.

Sorbitan olivate is commercially available, for example, as Olivem^{®} 900 (INCI Name: Sorbitan Olivate), which is a PEG-free co-emulsifier, an ivory waxy solid in flake form with melting range 65 - 75°C.

Microcrystalline wax is commercially available, for example, as Microcrystalline Wax P (INCI Name: Microcrystalline Wax & Tocopherol).

Dextrin palmitate is commercially available, for example, Rheopearl KL2 (INCI Name: Dextrin Palmitate).

Amounts of sorbitan olivate, microcrystalline wax and dextrin palmitate in the composition may vary. For example, in some embodiments, the composition may contain from about 0.05 mass % to about 1 mass % or from about 0.1 mass % to about 1 mass % of the sorbitan olivate; from about 0.01 mass % to about 1 mass % or from about 0.05 mass % to about 1.0 mass % of the microcrystalline wax and from about 2 mass % to about 10 mass % or about 3 mass % to about 10 mass % or from about 3 mass % to 8 mass % of the dextrin palmitate.

### Bentone

Disteardimonium hectorite may be a part of a thickening system of the composition.

In some embodiments, disteardimonium hectorite (bentone) may be used in a form of a gel already pre-mixed with a solvent. One non-limiting example of such gel is BENTONE GEL ISD V (INCI: ISODODECANE & DISTEARDIMONIUM HECTORITE & PROPYLENE CARBONATE).

In some embodiments, disteardimonium hectorite organoclay may be in a form of a powder.

In some embodiments, disteardimonium hectorite may be Bentone^{®} 38, which an organic derivative of a hectorite clay, in a powder form commercially available from Elementis Specialties Inc. I

An amount of disteardimonium hectorite organoclay in the composition may vary. In some embodiments, a content of disteardimonium hectorite organoclay in a powder form, such as disteardimonium hectorite, may be from 0.1 mass % to 5 mass % or from 0.1 mass % to 3 mass % or from 1 mass % to 3 mass % or from 0.1 mass % to 2 mass % or from 0.5 mass % to 2 mass % or from 1 mass % to 2 mass % or about 2 mass % or any value or subrange within these ranges. The disteardimonium hectorite can come from a powder or from a gel.

### Powders

One or more powders may form a powder phase of the composition.

The one or more powders may include mica, including natural mica and/or synthetic mica, such as synthetic fluorphlogopite. In some embodiments, the composition may include uncoated mica, which may include uncoated natural mica and/or uncoated synthetic mica, such as uncoated synthetic fluorphlogopite.

In some embodiments, the composition may include dimethicone coated mica, which may include dimethicone coated natural mica and/or dimethicone coated synthetic mica, such as dimethicone coated synthetic fluorphlogopite.

In some, embodiments, the composition may contain less than 10 % , less than 5 mass %, less than 2 mass %, less than 1 mass %, less than 0.5 mass % or less than 0.1 mass % of methicone coated powder, such as methicone coated mica, including methicone coated natural mica and/or methicone coated synthetic mica, such as methicone coated synthetic fluorphlogopite. The composition may contain no methicone coated powder, such as methicone coated mica, including methicone coated natural mica and/or methicone coated synthetic mica, such as methicone coated synthetic fluorphlogopite, at all.

In some embodiments, the composition may contain uncoated natural mica, uncoated synthetic mica, such as uncoated synthetic fluorphlogopite, and dimethicone coated synthetic mica, such as dimethicone coated synthetic fluorphlogopite.

In some embodiments, the one or more powders may also include silica, such as uncoated silica, and/or zinc myristate. In some embodiments, the one or more powders may include cellulose powder and/or cellulose derivative powder. In some embodiments, the one or more powder may include bismuth oxychloride powder, such as Ronaflair^{®} ESQ.

An amount of the one or more powders in the composition may vary. For example, in some embodiments, the composition may include from about 10 mass % to about 50 mass % or from about 10 mass % to about 45 mass % or from about 10 mass % to about 40 mass % or from about 20 mass % to about 40 mass % of the one or more powders.

### Oils

The composition may comprise at least one oil, which may form an oil phase of the composition.

In some embodiments, the at least one oil may comprise at least one hydrocarbon oil. In some embodiments, the at least one oil may comprise at least one silicone oil. In some embodiments, the at least one oil may include at least one hydrocarbon oil and at least one silicone oil.

In some embodiments, the at least one oil may comprise at least one oil solvent.

A non-polar oil may be one or more of non-polar oils disclosed in U.S. Patent No. 10,154,954, which is incorporated herein by reference it its entirety.

Nonpolar oils are usually hydrocarbons. They lack an electron-egative element like oxygen, which results in their typical hydrocarbon feel.

These oils may be of vegetable, mineral or synthetic origin.

The term "non-polar oil" may mean an oil for which the solubility parameter at 25 degrees centigrade, deltaa, as defined in U.S. Patent No. 10,154,954 is equal to 0 (J/cm³)^{1/2}.

The term "hydrocarbon oil" may mean an oil formed essentially from, indeed even composed of, carbon and hydrogen atoms, and optionally oxygen and nitrogen atoms, and not containing any silicon or fluorine atoms. It may contain alcohol, ester, ether, carboxylic acid, amine and/or amide groups.

In some embodiments, the non-polar oil may include one or more non-volatile non-polar hydrocarbon oils.

The non-volatile non-polar hydrocarbon oil may be chosen from linear or branched hydrocarbons of mineral or synthetic origin, such as: liquid paraffin or derivatives thereof, squalane, isoeicosane, naphthalene oil, alkane; polybutylenes such as Indopol H-100 (molar mass or MW=965 g/mol), Indopol H-300 (MW=1340 g/mol) and Indopol H-1500 (MW=2160 g/mol) sold or manufactured by the company Amoco, hydrogenated polyisobutylenes such as Parleam(R) sold by the company Nippon Oil Fats Corporation, Panalane H-300 E sold or manufactured by the company Amoco (MW=1340 g/mol), Viseal 20000 sold or manufactured by the company Synteal (MW=6000 g/mol) or Rewopal PIB 1000 sold or manufactured by the company Witco (MW=1000 g/mol), decene/butene copolymers, polybutene/polyisobutene copolymers, especially Indopol L-14, polydecenes and hydrogenated polydecenes such as: Puresyn 10 (MW=723 g/mol) and Puresyn 150 (MW=9200 g/mol) sold or manufactured by the company Mobil Chemicals, and mixtures thereof.

In some embodiments, the at least one non-polar oil is chosen from hydrogenated polyisobutenes and/or polybutenes.

In some embodiments, the at least one non-polar oil may include squalane (a triterpene consisting of 2,6,10,15,19,23-Hexamethyltetracosane).

In some embodiments, the at least one non-polar oil may include C₉-C₁₄ alkanes, such as isoparaffins, isododecane and isohexadecane. In some embodiments, the at least one non-polar oil may include Vegelight C912-LC, which is C₉₋₁₂ Alkanes (and) Coco-caprylate/caprate.

In some embodiments, the at least one oil solvent may comprise at least volatile oil solvent and at least one non-volatile oil solvent. In some embodiments, the at least one oil solvent may comprise a volatile hydrocarbon oil and a non-volatile hydrocarbon oil.

A volatile oil, such a volatile hydrocarbon oil, refers to an oil, such as hydrocarbon oil, having a boiling point lower than 300° C at 1 atm. Examples of volatile hydrocarbon oils include, but not limited to, C₉-C₁₄ alkanes, such as isoparaffins, isododecane and isohexadecane. In some embodiments, a volatile hydrocarbon oil may be Vegelight C912-LC, which is C₉₋₁₂ Alkanes (and) Coco-caprylate/caprate.

A non-volatile oil, such as non-volatile hydrocarbon oil, refers to an oil, such as a hydrocarbon oil, having a boiling point of 300°C. or higher at 1 atm, Examples of non-volatile hydrocarbon oils include, but not limited to, light squalane, hydrogenated polydecene and vaseline.

A non-limiting example of a silicone oil is dimethicone.

In some embodiments, the at least one oil may including at least one C₉-C₁₄ alkane, such as isoparaffins, isododecane and isohexadecane, and at least one silicone oil, such as dimethicone.

A content of the at least one oil in the composition may vary. For example, in some embodiments, the composition may include from about 10 mass % to about 50 mass % or from about 10 mass % to about 45 mass % or from about 10 mass % to about 40 mass % or from about 20 mass % to about 40 mass % of the at least one oil.

### Additional Ingredients

The composition may comprise one or more ingredients in addition to those discussed above. Such additional ingredients may include vitamins, such as tocopheryl acetate; a film former, such as trimethylsiloxysilicate; preservatives, such as phenoxythanol; emollients, such as cetyl ethylhexanoate and/or caprylic/capric triglyceride; ferments; extracts; pigments, such as titanium dioxide. An amount of the one or more ingredients may vary. For example, the composition may be from 1 mass % to 20 mass %.

### Methods of making

The composition may be prepared as follows. First, ingredients of Phase A, which may include one or more oils, may be mixed in a first "main" container. The ingredients of Phase A may include a hydrocarbon oil, such as isododecane, and a silicone oil, such as dimethicone. The ingredients of Phase A may further include one or more vitamins, such as tocopheryl acetate; a film former, such as trimethylsiloxysilicate; one or more preservatives, such as phenoxythanol; and/or one or more extracts. Second, ingredients of Phase B0, e.g. a hydrocarbon oil, e.g. isododecane; ingredients of Phase B1, such as disteardimonium hectorite; and/or ingredients of Phase B2, such as pigments, e.g. titanium dioxide pigments may be added to a second "side" container and dispersed until uniform. Once uniform, the ingredients of Phases B0, B1 and/or B2 may be added to the first "main" container. Third, component(s) of Phase D, e.g. one or more powders, may be added to the first "main" container one at a time and dispersed until uniform. Fourth, the silicone elastomer may be added to the first "main" container, dispersed until uniform, while heating the first "main" container to an elevated temperature, such as 45 to 50 °C. Fifth, ingredients of Phase C, which may include waxes, which may include sorbitan olivate, microcrystalline wax and dextrin palmitate may be added to a third "side" container. The ingredients of Phase C may also include one or more emollients, such as cetyl ethylhexanoate and/or caprylic/capric triglyceride. The ingredients of Phase C may be heated in the third "side" container to an elevated temperature, such as 80 to 85°C. Then, the ingredients of Phase C may be added to the first "main" container and dispersed until uniform.

### Exemplary Compositions

One exemplary composition may contain from about 9 mass % to about 11.5 mass % of dimethicone crosspolymer; from about 0.05 mass % to about 1 mass % of the sorbitan olivate; from about 0.01 mass % to about 1 mass % of the microcrystalline wax; from about 3 mass % to about 10 mass of the dextrin palmiate; about 2 mass % of the disteardimonium hectorite; from about 10 mass % to about 40 mass% of at least one powder and from about 10 mass % to about 40 mass % of at least one oil, while containing neither a volatile cyclic silicone, e.g. volatile cyclomethicone (D4-D6) silicone solvent, such as decamethylcyclopentasiloxane, nor talc.

Embodiments described herein are further illustrated by, though in no way limited to, the following working examples.

### EXAMPLE

Materials used in this Example include:

### Waxes

Trade Name: OLIVEM^{®} 900. INCI NAME: SORBITAN OLIVATE
Trade Name: MICRO CRYSTALLINE WAX P. INCI NAME: MICROCRYSTALLINE WAX & TOCOPHEROL
Trade Name: RHEOPEARL KL2. INCI Name: DEXTRIN PALMITATE

### Silicone Elastomer

Trade Name: DOWSIL^{™} EL-9241 DM SILICONE ELASTOMER BLEND. INCI Name: DIMETHICONE & DIMETHICONE CROSSPOLYMER

### Bentone Powder

Trade Name: BENTONE 38VCG. INCI Name: DISTEARDIMONIUM HECTORITE
Table 1 provides ingredients of an exemplary composition.

**Table 1**

| Raw Material/Trade Name | INCI Name | % |
|---|---|---|
| PERMETHYL 99A | ISODODECANE | 26.000000 |
| PDM-15 (DS) | SYNTHETIC FLUORPHLOGOPITE & DIMETHICONE | 14.500000 |
| SERICITE CS-15 | MICA | 5.000000 |
| DM-FLUID-2CS (KF-96A-2CS | DIMETHICONE | 3.000000 |
| RONAFLAIR ESQ | BISMUTH OXYCHLORIDE | 3.000000 |
| DM-FLUID-1.5CS (KF-96L-1.5CS) | DIMETHICONE | 3.000000 |
| SALACOS 816T | CETYL ETHYLHEXANOATE TITANIUM DIOXIDE & ISOPROPYL TITANIUM | 4.000000 |
| UNIPURE WHITE LC 987 ADT-C | TRIISOSTEARATE & BIS-PEG-15 DIMETHICONE/IPDI COPOLYMER & PEG-2 | 0.350000 |
| O.D.O | SOYAMINE CAPRYLIC/CAPRIC TRIGLYCERIDE | 3.000000 |
| PHENOXETOL NF 272556 | PHENOXYETHANOL | 0.200000 |
| VITAMIN E ACETATE | TOCOPHERYL ACETATE | 0.200000 |
| **OLIVEM 900** | **SORBITAN OLIVATE** | **0.200000** |
| **BENTONE 38VCG** | **DISTEARDIMONIUM HECTORITE** | **2.000000** |
| MSS-500W | SILICA | 2.000000 |
| ZINC MYRISTATE (POWDER BASE M) | ZINC MYRISTATE | 3.000000 |
| **REOPEARL KL2 (CHIBA)** | **DEXTRIN PALMITATE** | **5.000000** |
| RIFERM-BS | SACCHAROMYCES/RICE FERMENT FILTRATE & BUTYLENE GLYCOL & PHENOXYETHANOL & ETHYLHEXYLGLYCERIN & POTASSIUM SORBATE | 0.100000 |
| **MICRO CRYSTALLINE WAX P** | **MICROCRYSTALLINE** WAX & **TOCOPHEROL** | **0.350000** |
| NATIVE ESSENCE^{™} | CAPRYLIC/CAPRIC TRIGLYCERIDE & CRITHMUM MARITIMUM EXTRACT | 0.100000 |
| **DOWSIL^{™} EL-9241 DM SILICONE ELASTOMER BLEND** | **DIMETHICONE & DIMETHICONE CROSSPOLYMER** | **10.000000** |
| PDM-1000 | SYNTHETIC FLUORPHLOGOPITE | 7.500000 |
| BELSIL^{®} TMS 803 | TRIMETHYLSILOXYSILICATE | 7.500000 |

Table 2 provides ingredients of the composition of Table 1 distributed by phases.

**Table 2.**

| ***Phase*** | ***RM Name*** | ***EU INCI*** | ***RM %*** |
|---|---|---|---|
| A | PERMETHYL 99A | ISODODECANE | 20.000000 |
| A | BELSIL^{®} TMS 803 | TRIMETHYLSILOXYSILICATE | 7.500000 |
| A | DM-FLUID-2CS (KF-96A-2CS) | DIMETHICONE | 3.000000 |
| A | DM-FLUID-1.5CS (KF-96L-1.5CS) | DIMETHICONE | 3.000000 |
| A | VITAMIN E ACETATE | TOCOPHERYL ACETATE | 0.200000 |
| A | MICROCARE^{®} PE | PHENOXYETHANOL SACCHAROMYCES/RICE FERMENT | 0.200000 |
| A | RIFERM-BS | FILTRATE, BUTYLENE GLYCOL, PHENOXYETHANOL, ETHYLHEXYLGLYCERIN, POTASSIUM SORBATE | 0.100000 |
| A | NATIVE ESSENCE^{™} | CAPRYLIC/CAPRIC TRIGLYCERIDE, CRITHMUM MARITIMUM EXTRACT | 0.100000 |
| B0 | PERMETHYL 99A | ISODODECANE | 6.000000 |
| B1 | BENTONE 38VCG | **DISTEARDIMONIUM HECTORITE** | **2.000000** |
| B2 | UNIPURE WHITE LC 987 ADT-C | CI 77891, ISOPROPYL TITANIUM TRIISOSTEARATE, BIS-PEG-15 DIMETHICONE/IPDI COPOLYMER, PEG-2 SOYAMINE | 0.350000 |
| B3 | DOWSIL^{™} EL-9241 DM SILICONE ELASTOMER BLEND | **DIMETHICONE, DIMETHICONE CROSSPOLYMER** | **10.000000** |
| C0 | SALACOS 816T | CETYL ETHYLHEXANOATE | 4.000000 |
| C0 | O.D.O. | CAPRYLIC/CAPRIL TRIGLYCERIDE | 3.000000 |
| C1 | **OLIVEM 900** | **SORBITAN OLIVATE** | **0.200000** |
| C1 | **MICRO CRYSTALLINE WAX P** | **CERA MICROCRISTALLINA, TOCOPHEROL** | **0.350000** |
| C1 | **REOPEARL KL2 (CHIBA)** | **DEXTRIN PALMITATE** | **5.000000** |
| D | RONAFLAIR ESQ | CI 77163 | 3.000000 |
| D | SERICITE CS-15 | MICA | 5.000000 |
| D | PDM-15 (DS) | SYNTHETIC FLUORPHLOGOPITE, DIMETHICONE | 14.500000 |
| D | PDM-1000 | SYNTHETIC FLUORPHLOGOPITE | 7.500000 |
| D | ZINC MYRISTATE (POWDER BASE M) | ZINC MYRISTATE | 3.000000 |
| D | MSS-500W | SILICA | 2.000000 |
| | | | **100.000000** |

The composition was prepared as follows: (1) In the main container, the components of phase A were mixed. (2) Components of phases B0, B1, and B2 , were added in a side beaker and dispersed until unform. Once uniform, the components of phases B0, B1 and B2 were added to the main container. (3) Phase D components (powders) were added one at a time to the main container and dispersed until uniform. (4) Phase B3 was added to the main container, dispersed until uniform and the main container was heated to 45-50 °C. (5) Phases C0 and C1 were mixed in a side beaker, and heated to 80-85°C. The mixed phased C0 and C1 were then added to the main container and dispersed until uniform.

Table 3 examines an effect of an amount of silicone elastomer (DOWSIL^{™} EL-924) on a texture of a composition. Composition FL-0 in Table 3 is the composition of Tables 1 and 2.

**Table 3**

| | | | | | | |
|---|---|---|---|---|---|---|
| Raw Material/Trade INCI Name | INCI Name | FL-1 | FL-2 | FL-3 | FL-4 | FL-0 |
| PERMETHYL 99A | ISODODECANE | 22.000000 | 24.500000 | 27.500000 | 26.500000 | 26.000000 |
| GANEX^{™} V-220F (ANTARON ^{™} V-220F) | VP/EICOSENE COPOLYMER | 6.500000 | 7.000000 | 7.000000 | 5.000000 | |
| OLIVEM 900 | SORBITAN OLIVATE | 0.200000 | 0.200000 | 0.200000 | 0.200000 | 0.200000 |
| BENTONE 38VCG | DISTEARDIMONIU M HECTORITE | 2.000000 | 2.000000 | 2.000000 | 2.000000 | 2.000000 |
| REOPEARL KL (CHIBA) | DEXTRIN PALMITATE | 5.000000 | 5.000000 | 5.000000 | 5.000000 | 5.000000 |
| MICRO CRYSTALLI | MICROCRYSTALL NE WAX & | 0.350000 | 0.350000 | 0.350000 | 0.350000 | 0.350000 |
| NE WAX P DOWSIL^{™} | TOCOPHEROL | | | | | |
| EL-9241 DM SILICONE | DIMETHICONE & DIMETHICONE | 15.000000 | 12.000000 | 9.000000 | 10.000000 | 10.000000 |
| ELASTOMER BLEND | CROSSPOLYMER | | | | | |
| BELSIL^{®} TMS 803 | TRIMETHYLSILOX YSILICATE | | | | 2.000000 | 7.500000 |
| | Texture | Too Thick, hard to move/sprea d | Too Thick, hard to move/sprea d max value of elastomer 11.5 | Not viscous enough but can be min value of elastomer | Better texture but still doesn't stay put | acceptable |

All the compositions of Table 3 were prepared the same way as the composition of Tables 1-2.

Table 3 shows that levels of the silicone elastomer between 9% and 11.5% provide a better texture. FL-1 with 15% elastomer made it difficult to move during batching. FL-2 with 12% was too thick. FL-3 with 9% elastomer was not viscous enough. FL-4 with 10% elastomer was better than other compositions for texture, cushion and blurring. This level of elastomer was used in FL-0.

Table 4 evaluates an effect of varying amounts of the three waxes: sorbitan olivate, microcrystalline wax and dextrin palmitate.

**Table 4.**

| | | | | | | | | |
|---|---|---|---|---|---|---|---|---|
| Raw Material/ Trade Name | INCI Name | FL-0 | FL-5 | FL-6 | FL-7 | FL-8 | FL-9 | FL-10 |
| OLIVEM 900 | SORBITAN OLIVATE | 0.200 00 | 0.0500 00 | 1.00000 0 | 0.200000 | 0.200000 | 0.2000 00 | 0.2000 00 |
| BENTONE 38VCG | DISTEARDIMON IUM HECTORITE | 2.0000 00 | 2.0000 00 | 2.00000 0 | 2.000000 | 2.000000 | 2.0000 00 | 2.0000 00 |
| REOPEARL KL (CHIBA) | DEXTRIN PALMITATE | 5.0000 00 | 5.0000 00 | 5.00000 0 | 5.000000 | 5.000000 | 8 | 2.0000 00 |
| MICROCRYSTALLLLINEINE WAXMICROCRYSTAWAX &PTOCOPHEROL MICRO | | 0.3500 00 | 0.3500 00 | 0.35000 0 | 1.000000 | 0.100000 | 0.3500 00 | 0.3500 00 |
| DOWSIL^{™} EL-9241 DM SILICONE ELASTOM ER BLEND | DIMETHICONE & DIMETHICONE CROSSPOLYME R | 10.000 000 | 10.000 000 | 10.0000 00 | 10.000000 | 10.00000 | 10.000 000 | 10.000 000 |
| | Texture | accepta ble | Reduce d sorbitan olivate QS with IDD. Min value | Max value of sorbitan olivate QS with IDD Accepta ble | Max level of Microcrysta lline wax Acceptable | Min Microcryst aline wax QS from IDD Not Acceptable | Reopea rl Max Not acceptable | Reopea rl Min Not accepta ble |

The data in Table 4 indicates as follows:
(a) A desirable level for sorbitan olivate wax may be between 0.05% and 1%. Below 0.05% significantly reduced the cushion of the texture and above 1%, the formula became too thick.
(b) A desirable level for dextrin palmitate wax may be between 2% and 8%. At 2% and below the formula becomes unstable. At 8% and above the texture changes drastically.
(c) A desirable level for microcrystalline wax may be between 0.1% and 1%. At 0.1% and below the texture becomes liquid-like and above 1% the formula was unstable.

Table 5 provides results of evaluation of shear viscosities, hardness and texture for compositions with different levels of waxes.

**Table 5**

| **Wax Name** | MICROCRYSTALLINE | | |
|---|---|---|---|
| | SORBITAN OLIVATEWAX & TOCOPHEROL | | DEXTRIN PALMITATE |
| **Max % / Min %** | 1.0%/0.05% | 1.0%/.01% | 8%/2% |
| **Inventive/optimal levels** | 0.2% | 0.35% | 5% |
| **Hardness (g)** | 25/31 | 26/20 | 43/19 |
| **Viscosity (Pa.s) at sheer rate of 90(1/s)** | 6.27/7.43 | 6.07/5.07 | 9.76/3.79 |
| **Viscosity (Pa.s) at sheer rate of 4(1/s)** | 127.28/144.98 | 113.32/88.43 | 206.47/52.54 |
| **Texture** | **Min is smooth but with less cushion** | **Min is light and smooth but with too much slip/playtime** | **Biggest difference in texture between max/min values.** |
| **Stability** | **Stable** | **Stable** | **Min Not Stable** |

Adjusting dextrin palmitate was challenging because texture fluctuated a lot at each level. The minimum range was not stable due to excess sweating while the max range was too heavy. Sorbitan Olivate and Microcrystalline wax range proved to be comparable to standard with minimum differences in texture.

### Methods of testing:

Hardness- Rheo Meter Compac-100II, Test Method: 8mm, 10 mm depth, 300 mm/min
Viscosity -Rheometer DHRH20, RT cone .1 to 1000 sheer rate
Texture- Rubbed on skin
Stability- Visual test of COAT (color, odor, appearance, textures)

The figure shows a shear profile (a plot of viscosity in Pa·s versus a shear rate in s⁻¹). The figure shows that compositions FL-5 through FL-10 show 21.6-80.0% less in viscosity at low shear rates compared to FL-0. FL-5, FL-6, FL-7, FL-8, and FL-10 are 16.9% - 57.6% lower in viscosity at high shear rate compared to FL-0. FL-9 is 6.9% higher in viscosity at high shear rate compared to FL-0. FL-10 has the lowest viscosity profile (most shear thinning) out of all samples. The acceptable shear viscosity range may be 1000 - 20000 Pa·s at .01(1/s) and 1 - 100 Pa·s at 1000 (1/s).

Table 6 examines an effect of an amount of bentone powder (disteardimonium hectorite) on texture.

**Table 6**

| Raw Material | INCI Name | FL-11 | FL-12 | FL-0 |
|---|---|---|---|---|
| BENTONE GEL | ISODODECANE & DISTEARDIMONIUM | | | |
| ISD V #10932 | HECTORITE & PROPYLENE CARBONATE | 34.000000 | | |
| OLIVEM 900 | SORBITAN OLIVATE | 0.200000 | 0.200000 | 0.200000 |
| BENTONE 38VCG | DISTEARDIMONIUM HECTORITE | | 2.000000 | 2.000000 |
| REOPEARL KL (CHIBA) | DEXTRIN PALMITATE | | 5.000000 | 5.000000 |
| MICRO | MICROCRYSTALLINE | | | |
| CRYSTALLINE WAX P | WAX & TOCOPHEROL | 0.350000 | 0.350000 | 0.350000 |
| DOWSIL^{™} EL-9241 DM SILICONE | DIMETHICONE & DIMETHICONE | | 15.000000 | 10.000000 |
| ELASTOMER | CROSSPOLYMER | | | |
| BLEND | Texture | Soft, oil kicking out | Slightly heavy, hard to move/spread due to increased elastomer content | Optimized |

The data in Table 6 shows that 2% of Bentone (DISTEARDIMONIUM HECTORITE) is a workable level providing a desirable texture.

FL-11 used Bentone Gel with about 7% Bentone powder mixed with 27% IDD, yielding 4.55% of disteardimonium hectorite. The result displayed soft and oily texture that was not desirable.

FL-12 and FL-0 used just 2% of bentone powder without the Gel, yielding 2% of disteardimonium hectorite. This amount provided a good amount of slip, cushion, and a desirable texture.

Table 7 provides ingredients for exemplary composition FL-13 containing methicone coated synthetic fluorphlogopite.

**Table 7**

| Raw Material | INCI Name | FL-13 |
|---|---|---|
| BENTONE GEL ISD V #10932 | ISODODECANE & DISTEARDIMONIUM | 15.000000 |
| | HECTORITE & PROPYLENE CARBONATE | |
| PERMETHYL 99A | ISODODECANE SYNTHETIC | 26.250000 |
| PDM-15 (DS) | FLUORPHLOGOPITE & DIMETHICONE | 10.000000 |
| KAOLIN ASP 400P | KAOLIN | |
| SERICITE CS-15 | MICA | 5.000000 |
| GANEX^{™} V-220F (ANTARON^{™} V-220F) | VP/EICOSENE COPOLYMER | 4.500000 |
| DM-FLUID-2CS (KF-96A-2CS) | DIMETHICONE | 5.000000 |
| RONAFLAIR ESQ | BISMUTH OXYCHLORIDE | 3.000000 |
| DM-FLUID-1.5CS (KF-96L-1.5CS) | DIMETHICONE | 4.000000 |
| SALACOS 816T | CETYL ETHYLHEXANOATE CAPRYLIC/CAPRIC | 5 .000000 |
| O.D.O | TRIGLYCERIDE | 4.000000 |
| PHENOXETOL NF 272556 | PHENOXYETHANOL | 0.200000 |
| VITAMIN E ACETATE (RM-06251) | TOCOPHERYL ACETATE | 0.200000 |
| OLIVEM 900 | SORBITAN OLIVATE | 0.200000 |
| BENTONE 38VCG | DISTEARDIMONIUM HECTORITE | 2.000000 |
| PDM-1000(S) | **SYNTHETIC FLUORPHLOGOPITE & METHICONE** | **10.000000** |
| UNIPURE WHITE LC 981 LL, 28372 | TITANIUM DIOXIDE & LAUROYL LYSINE | 0.100000 |
| REOPEARL KL (CHIBA) | DEXTRIN PALMITATE | 5.000000 |
| RIFERM-BS | SACCHAROMYCES/RICE FERMENT FILTRATE & BUTYLENE GLYCOL & PHENOXYETHANOL & ETHYLHEXYLGLYCERIN & POTASSIUM SORBATE | 0.100000 |
| MICRO CRYSTALLINE WXMICROCRYSTALLINE WAX P | & TOCOPHEROL CAPRYLIC/CAPRIC | 0.350000 |
| NATIVE ESSENCE^{™} | TRIGLYCERIDE & CRITHMUM MARITIMUM EXTRACT | 0.100000 |

FL-13 composition turned purple in color, which is undesirable.

Although the foregoing refers to particularly preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

## Claims

1. An anhydrous cosmetic composition comprising (a) one or more oils; (b) a thickening system comprising a silicone elastomer, disteardimonium hectorite and waxes; and (c) one or more powders, said waxes comprise sorbitan olivate, microcrystalline wax and dextrin palmitate.

2. The cosmetic composition of claim 1, wherein a combined amount of the sorbitan olivate, the microcrystalline wax and the dextrin palmitate in the composition is from about 3 mass % to about 12 mass %.

3. The cosmetic composition of claim 1 or 2, wherein the silicone elastomer comprises dimethicone crosspolymer.

4. The cosmetic composition of claim 3, wherein an amount of the dimethicone crosspolymer in the composition is from about 9 mass % to about 11.5 mass %.

5. The cosmetic composition of any one of the preceding claims comprising from about 0.05 mass % to about 1 mass % of the sorbitan olivate; from about 0.01 mass % to about 1 mass % of the microcrystalline wax and from about 3 mass % to about 10 mass of the dextrin palmitate.

6. The cosmetic composition of any one of the preceding claims comprising from about 1 mass % to about 3 mass % of the disteardimonium hectorite.

7. The cosmetic composition of claim 6 comprising about 2 mass % of the disteardimonium hectorite.

8. The cosmetic composition of any one of the preceding claims, wherein an amount of the one or more powders in the composition is from about 10 mass % to about 40 mass %.

9. The cosmetic composition of any one of the preceding claims, wherein the one or more powders comprise mica.

10. The cosmetic composition of any one of the preceding claims, wherein an amount of the one or more oils in the composition is from about 10 mass % to about 40 mass %.

11. The cosmetic composition of any one of the preceding claims, wherein the one or more oils comprise isododecane; and/or
wherein the composition does not contain a volatile cyclic silicone; and/or
wherein the composition does not contain talc; and/or
wherein the composition does not contain methicone coated mica.

12. The cosmetic composition of any one of the preceding claims comprising from about 9 mass % to about 11.5 mass % of dimethicone crosspolymer; from about 0.05 mass % to about 1 mass % of the sorbitan olivate; from about 0.01 mass % to about 1 mass % of the microcrystalline wax; from about 3 mass % to about 10 mass of the dextrin palmiate; about 2 mass % of the disteardimonium hectorite; from about 10 mass % to about 40 mass% of at least one powder and from about 10 mass % to about 40 mass % of at least one oil, wherein the composition does not contain either a volatile cyclic silicone, or talc.

13. The cosmetic composition of any one of the preceding claims, wherein the composition has a shear viscosity at 0.01 s⁻¹ from about 1000 Pa·s to about 20000 Pa·s; and/or wherein the composition has a shear viscosity at 1000 s⁻¹ from about 1 Pa·s to about 100 Pa·s.

14. The cosmetic composition of any one of the preceding claims, wherein the cosmetic composition is an eyeshadow base composition.

15. A cosmetic method comprising applying the cosmetic composition of any one of the preceding claims to a keratinous surface.
